(19) Europäisches Patentamt
European Patent Office
Office européen des brevets

(11) Veröffentlichungsnummer: **0 048 373**
**B1**

(12) # EUROPÄISCHE PATENTSCHRIFT

(45) Veröffentlichungstag der Patentschrift: **09.05.84**

(51) Int. Cl.³: **C 07 D 231/12**

(21) Anmeldenummer: **81107014.3**

(22) Anmeldetag: **07.09.81**

(54) **Verfahren zur Herstellung von Pyrazol.**

(30) Priorität: **19.09.80 DE 3035395**

(43) Veröffentlichungstag der Anmeldung: **31.03.82 Patentblatt 82/13**

(45) Bekanntmachung des Hinweises auf die Patenterteilung: **09.05.84 Patentblatt 84/19**

(84) Benannte Vertragsstaaten: **BE CH DE FR GB IT LI NL**

(56) Entgegenhaltungen:
**DE - A - 2 648 008**
**DE - A - 2 704 281**
**DE - B - 1 234 223**
**US - A - 2 515 160**

(73) Patentinhaber: **BAYER AG, Konzernverwaltung RP Patentabteilung, D-5090 Leverkusen 1 Bayerwerk (DE)**

(72) Erfinder: **Heinemann, Ulrich, Dr., Thorner Strasse 22, D-5600 Wuppertal 2 (DE)**
Erfinder: **Thomas, Rudolf, Dr., Dellbusch 269, D-5600 Wuppertal 2 (DE)**
Erfinder: **Lantzsch, Reinhard, Dr., Heymannstrasse 32, D-5090 Leverkusen 1 (DE)**
Erfinder: **Ditgens, Klaus, Dr., Claudiusweg 7, D-5600 Wuppertal 1 (DE)**
Erfinder: **Weber, Erhard, Dr., Claudiusweg 9, D-5600 Wuppertal 1 (DE)**

**EP 0 048 373 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues, vorteilhaftes Verfahren zur Herstellung von Pyrazol, welches als Ausgangsstoff zur Synthese von herbizid wirksamen Acetaniliden verwendet werden kann.

Es ist bereits bekannt geworden, dass man Pyrazol erhält, wenn man zunächst Hydrazinhydrat und Acrolein in Gegenwart von Ether als Verdünnungsmittel unter Kühlung zum 2-Pyrazolin umsetzt, dieses in Form seines Hydrochlorids isoliert, dann die Base in üblicher Weise freisetzt und anschliessend das 2-Pyrazolin in Gegenwart von Chloroform als Verdünnungsmittel mit Brom zum Pyrazol oxidiert (vergleiche Journal f.Praktische Chemie 50, 531 ff (1894)). Dieses Verfahren weist jedoch eine Reihe von Nachteilen auf. So muss bei der Herstellung des 2-Pyrazolins unter Kühlung gearbeitet werden. Ausserdem kann offensichtlich das 2-Pyrazolin aus dem Reaktionsgemisch nur in Form seines Hydrochlorids isoliert werden, welches zusätzlich noch in einem ausserordentlich aufwendigen Verfahren gereinigt werden muss. Hierbei wird das 2-Pyrazolinhydrochlorid in Ausbeuten um 50% der Theorie erhalten, die sich bei der anschliessend notwendigen Überführung des Salzes in die freie Base, deren weiterer Reinigung durch Extraktion und Destillation weiter erniedrigt.

Auch die Oxidation in der anschliessenden zweiten Stufe verläuft unbefriedigend, denn die Ausbeute beträgt nur etwa 40%. Hinzu kommt, dass das Brom, insbesondere für die Anwendung im technischen Massstab, kein ideales Oxidationsmittel darstellt.

Es wurde nun gefunden, dass sich Pyrazol aus Hydrazinhydrat und Acrolein herstellen lässt, wenn man Hydrazinhydrat mit Acrolein in Gegenwart eines aromatischen Kohlenwasserstoffes oder eines aromatischen Halogenkohlenwasserstoffes bei Temperaturen zwischen 20 und 80 °C umsetzt und das entstehende 2-Pyrazolin der Formel

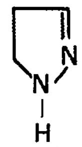

(I)

direkt, oder gegebenenfalls nach vorheriger Isolierung, mit Chlor oder Alkali- bzw. Erdalkali-hypochloriten in Wasser bzw. einem aromatischen Kohlenwasserstoff oder einem aromatischen Halogenkohlenwasserstoff, gegebenenfalls im Gemisch mit Wasser, bei Temperaturen zwischen 0 und 60 °C, gegebenenfalls in Gegenwart eines Katalysators, oxidiert.

Es ist als ausgesprochen überraschend zu bezeichnen, dass das erfindungsgemässe Verfahren unter den angegebenen Reaktionsbedingungen in guten Ausbeuten verläuft, denn aufgrund der Tatsache, dass Acrolein im Falle des bekannten Verfahrens selbst bei Kühlung teilweise polymerisiert (vgl. hierzu die oben genannten Literaturstelle), musste erwartet werden, dass beim Arbeiten ohne Kühlung eine verstärkte Polymerisation eintritt.

Die glatte Oxidation des Pyrazolins zum Pyrazol ist insofern auch überraschend, als dass sich ausschliesslich das gewünschte unsubstituierte Pyrazol bildet. Da Chlor eher zu Kernsubstitution neigt als Brom war die Bildung von chlorierten Pyrazolen, insbesondere 4-Chlorpyrazol, zu erwarten gewesen (vergleiche hierzu auch Liebigs Ann. der Chemie 598, 186 (1956)).

Das erfindungsgemässe Verfahren zeichnet sich durch eine Reihe von Vorteilen aus. So ist es mit Hilfe dieses Verfahrens möglich, Pyrazol in einfacher Weise, auch in technischem Massstab, mit hohen Ausbeuten herzustellen. Besonders hervorzuheben ist, dass praktisch keine Nebenprodukte auftreten und die Aufarbeitung keinerlei Schwierigkeiten bereitet. Ausserdem kann das als Zwischenprodukt anfallende Pyrazolin der Formel (I) in einfacher Weise isoliert werden, sofern letzteres gewünscht wird. Das erfindungsgemässe Verfahren stellt somit eine wertvolle Bereicherung der Technik dar.

Der Verlauf des erfindungsgemässen Verfahrens lässt sich durch das folgende Formelschema veranschaulichen:

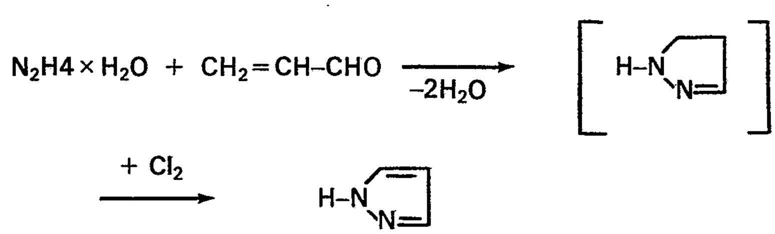


Als aromatische Kohlenwasserstoffe und aromatische Halogenkohlenwasserstoffe, die bei dem erfindungsgemässen Verfahren als Lösungsmittel fungieren, kommen vorzugsweise Benzol, Toluol, Xylol, Chlorbenzol und Dichlorbenzol in Frage.

Die Reaktionstemperaturen können bei der Durchführung des erfindungsgemässen Verfahrens in einem grösseren Bereich variiert werden. Im allgemeinen arbeitet man bei der Umsetzung des Hydrazinhydrats mit Acrolein bei Temperaturen zwischen 20 und 80 °C, vorzugsweise zwischen 20 und 60 °C. Bei der anschliessenden Oxidation des Pyrazolins der Formel (I) zum Pyrazol arbeitet man im allgemeinen bei Temperaturen zwischen 0 °C und 60 °C, vorzugsweise zwischen 0 °C und 30 °C.

Die Oxidation des 2-Pyrazolins zum Pyrazol

kann gegebenenfalls in Gegenwart eines Katalysators durchgeführt werden. Hierbei werden übliche Oxidationskatalysatoren verwendet, wie beispielsweise Rutheniumdioxid-monohydrat oder Rutheniumtrichlorid-monohydrat.

Bei der Durchführung des erfindungsgemässen Verfahrens setzt man vorzugsweise auf 1 Mol Hydrazinhydrat 1 bis 1,2 Mol Acrolein und auf 1 Mol entstehendes 2-Pyrazolin 1–2 Mol Chlor oder Alkali- bzw. Erdalkalihypochlorit, wie insbesondere Natrium-, Kalium- oder Calciumhypochlorit, ein.

Das erfindungsgemässe Verfahren wird im allgemeinen unter Atmosphärendruck durchgeführt. Es ist jedoch auch möglich, bei höheren oder niederen Drucken zu arbeiten.

Das als Zwischenprodukt entstehende 2-Pyrazolin kann gegebenenfalls isoliert werden, indem man das Reaktionsgemisch unter Rückfluss am Wasserabscheider erhitzt, um so das Wasser auszukreisen. Der Rückstand wird im Vakuum eingeengt. Das erhaltene rohe 2-Pyrazolin kann durch weitere Destillation im Vakuum über eine Kolonne gereinigt werden.

Das Pyrazolin kann in reiner Form, in Form seiner organischen Lösung nach dem Wasserauskreisen, in Wasser oder aber direkt in wässrig-organischer Lösung weiter umgesetzt werden.

Im einzelnen verfährt man bei der Durchführung des erfindungsgemässen Verfahrens so, dass man Hydrazinhydrat in einem aromatischen Kohlenwasserstoff oder einem aromatischen Halogenkohlenwasserstoff mit Acrolein versetzt und nach dem Abklingen der stark exothermen Reaktion entweder zunächst das Wasser aus dem Reaktionsgemisch entfernt (um daraufhin das Pyrazolin zu isolieren), oder aber dem Reaktionsgemisch unter Kühlung direkt das Oxidationsmittel zufügt. Die anschliessende Aufarbeitung erfolgt nach üblichen Methoden. Im allgemeinen verfährt man in der Weise, dass man das Reaktionsgemisch nach beendeter Oxidation mit wässriger Alkalibase verrührt, dann mehrfach mit einem mit Wasser wenig mischbaren organischen Lösungsmittel extrahiert, die vereinigten organischen Phasen trocknet und einengt. Das Pyrazol wird dabei in reiner Form erhalten.

Das nach dem erfindungsgemässen Verfahren herstellbare Pyrazol ist ein allgemein interessanter Synthesebaustein in der organischen Chemie. Insbesondere kann Pyrazol als Ausgangsstoff zur Synthese von herbizid wirksamen Acetaniliden dienen (vgl. DE-OS 26 48 008 und DE-OS 27 04 281).

So lässt sich beispielsweise das 2,6-Diethyl-N-(pyrazol-1-yl-methyl)-chloracetanilid herstellen, indem man 2,6-Diethyl-N-chlormethyl-chloracetanilid in Gegenwart eines Verdünnungsmittels sowie in Gegenwart eines Säurebinders mit Pyrazol umsetzt. Formelmässig lässt sich diese Synthese wie folgt wiedergeben:

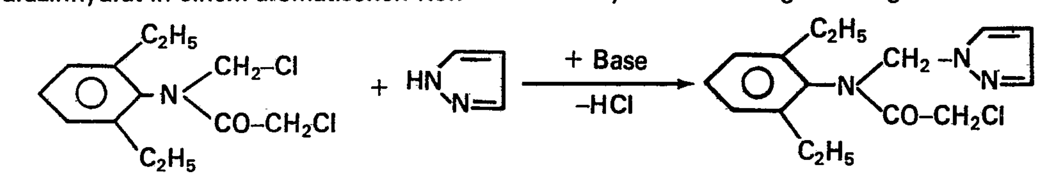


Das erfindungsgemässe Verfahren wird durch die nachfolgenden Beispiele veranschaulicht.

Herstellungsbeispiele

Beispiel 1
(mit Isolierung des 2-Pyrazolins)

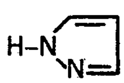

a) 200 g (4 Mol) Hydrazinhydrat in 600 ml Toluol werden unter Rühren mit 268,8 g (4,8 Mol) Acrolein versetzt. Nach Abklingen der stark exothermen Reaktion (ca. 60 °C) wird das Reaktionsgemisch unter Rückfluss am Wasserabscheider erhitzt, bis sich die berechnete Menge Wasser (144 ml) abgeschieden hat. Die zurückbleibende Lösung wird durch Abdestillieren des Toluols im Vakuum eingeengt. Das erhaltene Rohprodukt wird nochmals im Vakuum über eine Kolonne destilliert. Man erhält 227 g (81% der Theorie) 2-Pyrazolin als farbloses Öl vom Siedepunkt 50 °C/24 mbar.

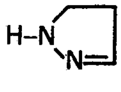

b) Zu 24,4 g (0,35 Mol) des so erhaltenen 2-Pyrazolins in 500 ml Toluol werden unter Eiskühlung und kräftigem Rühren 24,7 g (0,35 Mol) Chlor eingeleitet. Man lässt 30 Minuten rühren, engt das Reaktionsgemisch ein und versetzt den Tückstand mit 280 ml 20%iger wässriger Natronlauge. Man lässt erneut 30 Minuten rühren und extrahiert anschliessend fünfmal mit je 500 ml Ether. Die vereinigten organischen Phasen werden über Natriumsulfat getrocknet und eingeengt. Man erhält 17 g (72% der Theorie) Pyrazol vom Schmelzpunkt 69–70 °C.

c) Zu 7 g (0,1 Mol) des gemäss a) erhaltenen 2-Pyrazolins in 22 ml Wasser werden bei 10–25 °C (Eiskühlung) unter kräftigem Rühren 12 g (0,17 Mol) Chlor eingeleitet. Man lässt 30 Minuten bei Raumtemperatur nachrühren und versetzt mit 16 g (0,4 Mol) Natriumhydroxid in 44 ml Wasser. Anschliessend wird viermal mit je 75 ml Methylenchlorid extrahiert. Die vereinigten organischen Phasen werden über Natriumsulfat getrocknet und im Vakuum eingeengt. Das zurückbleibende Öl kristallisiert rasch durch. Man erhält 4,9 g (72% der Theorie) Pyrazol vom Schmelzpunkt 68–69 °C.

Beispiel 2
(ohne Isolierung des 2-Pyrazolins)

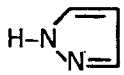

In eine entsprechend Beispiel 1 (Verfahrensschritt a) erhaltene Reaktionslösung von 70 g (1 Mol) 2-Pyrazolin (gaschromatographisch bestimmt) in Toluol/Wasser werden bei 10–25 °C (Eiskühlung) unter kräftigem Rühren 140 g (2 Mol) Chlor eingeleitet. Man lässt 30 Minuten bei Raumtemperatur nachrühren und engt im Vakuum ein. Nach Zugabe von 800 g 20%iger wässriger Natronlauge lässt man erneut 30 Minuten nachrühren. Anschliessend wird viermal mit je 1000 ml Methylenchlorid extrahiert, die vereinigten organischen Phasen werden über Natriumsulfat getrocknet und eingeengt. Der ölige Rückstand kristallisiert rasch durch. Man erhält 56 g (83% der Theorie) Pyrazol vom Schmelzpunkt 69 °C.

## Patentansprüche

1. Verfahren zur Herstellung von Pyrazol aus Hydrazinhydrat und Acrolein, dadurch gekennzeichnet, dass man Hydrazinhydrat mit Acrolein in Gegenwart eines aromatischen Kohlenwasserstoffes oder eines aromatischen Halogenkohlenwasserstoffes bei Temperaturen zwischen 20 und 80 °C umsetzt und das entstehende 2-Pyrazolin der Formel

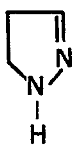
(I)

direkt, oder gegebenenfalls nach vorheriger Isolierung, mit Chlor oder Alkali- bzw. Erdalkali-hypochloriten in Wasser bzw. einem aromatischen Kohlenwasserstoff oder einem aromatischen Halogenkohlenwasserstoff, gegebenenfalls im Gemisch mit Wasser, bei Temperaturen zwischen 0 und 60 °C, gegebenenfalls in Gegenwart eines Katalysators, oxidiert.

2. Verfahren gemäss Anspruch 1, dadurch gekennzeichnet, dass man die Umsetzung von Hydrazinhydrat mit Acrolein bei Temperaturen zwischen 20 °C und 60 °C durchführt.

3. Verfahren gemäss Anspruch 1, dadurch gekennzeichnet, dass man die Oxidation des 2-Pyrazolins bei Temperaturen zwischen 0 °C und 30 °C durchführt.

4. Verfahren gemäss Anspruch 1, dadurch gekennzeichnet, dass man als Oxidationsmittel Chlor, Natriumhypochlorit, Kaliumhypochlorit oder Calciumhypochlorit einsetzt.

5. Verfahren gemäss Anspruch 1, dadurch gekennzeichnet, dass man als Katalysator Rutheniumdioxid-monohydrat oder Rutheniumtrichlorid-monohydrat einsetzt.

## Revendications

1. Procédé de fabrication de pyrazol à partir d'hydrate d'hydrazine et d'acroléine, caractérisé en ce qu'on fait réagir l'hydrate d'hydrazine avec l'acroléine en présence d'un hydrocarbure aromatique ou d'un hydrocarbure halogéné aromatique à des températures entre 20 et 80 °C et en ce qu'on oxyde la 2-pyrazoline obtenue de formule

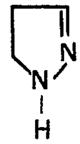
(I)

directement, ou éventuellement après isolation préalable, avec du chlore ou des hypochlorites alcalins ou alcalino-terreux dans de l'eau ou un hydrocarbure aromatique ou un hydrocarbure halogéné aromatique, éventuellement en mélange avec de l'eau, à des températures entre 0 et 60 °C, éventuellement en présence d'un catalyseur.

2. Procédé selon la revendication 1, caractérisé en ce qu'on effectue la réaction de l'hydrate d'hydrazine avec l'acroléine à des températures entre 20 °C et 60 °C.

3. Procédé selon la revendication 1, caractérisé en ce qu'on effectue l'oxydation de la 2-pyrazoline à des températures entre 0 °C et 30 °C.

4. Procédé selon la revendication 1, caractérisé en ce qu'on utilise comme agent oxydant du chlore, de l'hypochlorite de sodium, de l'hypochlorite de potassium ou de l'hypochlorite de calcium.

5. Procédé selon la revendication 1, caractérisé en ce qu'on utilise comme catalyseur du dioxyde de ruthénium monohydraté ou du trichlorure de ruthénium monohydraté.

## Claims

1. Process for the preparation of pyrazole from hydrazine hydrate and acrolein, characterised in that hydrazine hydrate is reacted with acrolein in the presence of an aromatic hydrocarbon or of an aromatic halogenated hydrocarbon at temperatures between 20 and 80 °C and the 2-pyrazoline formed, of the formula

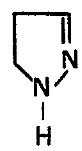
(I)

is oxidised directly, or where appropriate after first being isolated, with chlorine or alkali metal or alkaline earth metal hypochlorites in water or an aromatic hydrocarbon or an aromatic halogenated hydrocarbon, if appropriate mixed with water, at temperatures between 0 and 60 °C, if necessary in the presence of a catalyst.

2. Process according to Claim 1, characterised in that the reaction of hydrazine hydrate with acrolein is carried out at temperatures between 20 °C and 60 °C.

3. Process according to Claim 1, characterised in that the oxidation of 2-pyrazoline is carried out at temperatures between 0 °C and 30 °C.

4. Process according to Claim 1, characterised in that chlorine, sodium hypochlorite, potassium hypochlorite or calcium hypochlorite is used as the oxidising agent.

5. Process according to Claim 1, characterised in that ruthenium dioxide monohydrate or ruthenium trichloride monohydrate is used as the catalyst.